# NEW EUROPEAN PATENT SPECIFICATION

(11) **EP 3 437 513 B2**
(45) Date of publication and mention of the opposition decision: **02.10.2024**
(45) Mention of the grant of the patent: 02.12.2020
(21) Application number: 17775937.0
(22) Date of filing: 03.04.2017
(51) Int. Cl.: A61K 8/02, A45D 44/00, D04H 1/435, D04H 1/4334, D04H 3/005, A61K 8/85, A61K 8/88

(54) **NONWOVEN SHEET FOR FACIAL MASK**
VLIESSTOFF FÜR GESICHTSMASKE
FEUILLE NON TISSÉE DESTINÉE À UN MASQUE FACIAL

(30) Priority: 01.04.2016 KR 20160040222; 31.03.2017 KR 20170041593
(43) Date of publication of application: 06.02.2019
(73) Proprietor: Kolon Industries, Inc., Seoul 07793 (KR)
(72) Inventor: AHN, Tae Hwan, Gangseo-gu, Seoul 07793 (KR); KIM, Jin Il, Gangseo-gu, Seoul 07793 (KR); CHOI, Jin Hwan, Gangseo-gu, Seoul 07793 (KR); CHO, Hee Jung, Gangseo-gu, Seoul 07793 (KR)
(74) Representative: Ter Meer Steinmeister & Partner
(86) International application number: PCT/KR2017/003619
(87) International publication number: WO 2017/171513

(56) References cited:
- EP-A1- 0 455 927
- WO-A1-2010/064710
- CA-C- 2 575 662
- KR-A- 20060 013 495
- KR-A- 20090 001 093
- KR-A- 20110 015 029
- KR-B1- 100 711 182
- KR-B1- 101 579 419
- US-A- 5 899 785
- DATABASE WPI Week 201556, Derwent World Patents Index; AN 2015-47590H, XP002794859

## Description

### Technical Field

The present invention relates to a use of a nonwoven sheet as a mask pack sheet.

### Background Art

Many women have been interested in the beauty industry for a long period of time, and nowadays the beauty industry has become an industrial field in which many people are investing heavily for the purpose of self-care regardless of sex or age. Among the beauty fields, skin care in particular shares a long history with humankind dating back to ancient times. Skin care first originated in Rome and Greece, where women began to mix powder obtained by grinding grains such as barley and soybeans with honey, flowers, and vegetable juices, apply the mixture on their faces, and wash them with milk the next day.

In recent years, more and more people have become interested in beauty, and beauty products have become increasingly popular. Accordingly, skin-care methods and types for skin health, such as moisturizing of the skin, removing waste, whitening, and improving skin health, have developed in various manners. Among them, a mask pack is very popular among men as well as women because of its ability to make it easy to care for skin at home. Most of all, modern people live in a society where appearance is considered a competitive factor, and for busy modern people who cannot afford time for care, skin care using mask packs has become a way to save time and money while still obtaining satisfactory effects.

Mask packs are classified into various types, such as cream, gel, and wash-off types. Among the types, there is demand from many people for a sheet-type mask pack, which is manufactured so as to have functions such as whitening, alleviation of wrinkles, hydration, alleviation of skin problems, skin tightening, and antibacterial functions by wetting a sheet-type support in the shape of a human face with an essence containing nutritive substances such as natural extracts, proteins, and vitamins, because cosmetics are capable of coming into contact with the skin over a long period of time and because the pack is disposable and thus easy to use. In the sheet used as the support in the sheet-type mask pack, a nonwoven fabric manufactured using synthetic fibers such as polypropylene (PP), polyester (PET), and polyethylene (PE) is mostly used.

However, since the sheet manufactured based on the nonwoven fabric is not sufficiently adhered to the skin, there is a limitation in the ability to deliver an effective ingredient deeply within the skin. In order to solve this, an effort has been made to improve attachment to the skin by using an excessive amount of essence. However, the weight of the pack was increased, thus causing discomfort by flowing down from the face when used. Accordingly, in recent years, a hydrogel-type mask pack sheet characterized by good adhesion to the skin and thus facilitating daily activities even when a pack is worn has appeared and has become popular. However, the hydrogel mask pack sheet is heavy due to the great thickness thereof, and there is inconvenience in that the protective film attached in order to protect a gel portion must be removed for use.

Meanwhile, due to the popularity of the sheet-type mask pack, various related products and technologies have been developed. For example, Korean Patent Application KR 2011-0060525, entitled "Ultrafine melt-blown nonwoven fabric having good hygroscopicity and sheet mask for cosmetics using the same" introduces a technology for improving the absorbency of a mask sheet by using a hydrophilic additive on an ultrafine-yarn melt-blown nonwoven sheet. Korean Patent Application KR 2011-080066 entitled "Skin beauty sheet and method of manufacturing the same" proposes a skin mask pack which is on a two-layered nanofiber layer formed on a nonwoven fabric and which is surface-treated using a plasma.

However, the majority of the conventional technologies including the above-described technologies cannot completely solve problems such as weak contractility due to the limitation of the physical properties of the material constituting synthetic fibers, inconvenience of activity due to attachment to the skin, and frequent separation from the skin when the pack is worn for a long time. Further, there is a drawback in that since the strength of the nonwoven fabric manufactured using the melt-blown method is weak, self-supporting force is poor, whereby resistance to tearing is poor, and there is a limitation in that wettability with cosmetic compositions is also poor.
EP 2 301 380 A relates to a skin-covering sheet including a non-woven fabric in which a splittable conjugate fiber containing at least two components as viewed in fiber cross-section, namely a polyethylene component and another polymer component, and a hydrophilic fiber are blended, and hydroentangled.
JP 2015 148023 A is directed to a nonwoven fabric for a wet sheet substrate comprising splittable conjugate fibers, hydrophilic fibers and thick fineness synthetic fibers having fineness of 0.8 dtex or more.
WO 2010/064710 A relates to a nonwoven fabric which is formed by clustering fibers containing a thermoplastic elastomer on one side of a hydrophilic fiber layer.

### Disclosure

### Technical Problem

Accordingly, it is an object of the present invention to provide a use of a nonwoven sheet as a mask pack sheet, which has improved flexibility and wettability of a material and good self-supporting force and which is not easily torn compared to a conventional nonwoven-fabric type of mask pack sheet manufactured using synthetic fibers.

### Technical Solution

In order to accomplish the above object, the present invention provides a use of a nonwoven sheet as a mask pack sheet including split yarns formed from two or more types of thermoplastic resin, wherein the two or more types of thermoplastic resin include polyethylene terephthalate (PET) and nylon, wherein:
the split yarns are formed from the thermoplastic resin including 60 to 80 wt% polyethylene terephthalate (PET) and 20 to 40 wt% nylon.

The split yarns according to the embodiment may be one or more types selected from among eight-split yarns, sixteen-split yarns, and thirty-two-split yarns, and an average diameter per strand of fiber after being split may be 0.01 to 2 µm.

The nonwoven sheet according to the embodiment may be a spun-bond nonwoven fabric, and may have a unit weight of 30 to 60 g/m² on a dry-weight basis, a thickness of 0.15 to 0.3 µm, and a porosity of 60 to 95%.

### Advantageous Effects

According to the present invention, it is possible to improve adhesion and attachment to skin due to the good flexibility of a material and to improve wettability with respect to cosmetics, and it is also possible to secure self-supporting force and good strength of a nonwoven sheet due to the manufacture of a mask pack sheet using a spun-bond method.

### Best Mode

The present invention provides a use of a nonwoven sheet as a mask pack sheet including split yarns formed from two or more types of thermoplastic resin. Since the nonwoven sheet for the mask pack of the present invention includes fine-denier split yarns, porosity is high, and thus the sheet may satisfactorily keep a coarse cosmetic solution therein. Since flexibility is good, the sheet is adhered to the skin, thus exhibiting a good effect as a mask pack sheet.

The term "split yarn" of the present invention means a special fiber manufactured in a manner in which multiple strands of microfine fiber are separated from one filament after a plurality of types of polymer resin is subjected to complex spinning. The split yarn may be classified into various types according to the fiber section. A well-known representative split yarn is a sea-island yarn. However, the split yarn of the present invention may more closely approximate a radial type, exhibiting radial extension from the center of the fibers, like a spoke, and branching of fine-denier yarn, rather than the sea-island yarn.

The split yarn of the present invention is formed from two or more types of thermoplastic resin, thus exhibiting the characteristics of both types of thermoplastic resin. Particularly, when considering the contractility and chemical resistance of a mask pack sheet, the split yarn includes polyethylene terephthalate (PET). When considering the wearing sensation, such as the tactile sensation, the split yarn includes nylon. More preferably, since the split yarn is formed from two or more types of thermoplastic resin including polyethylene terephthalate (PET) and nylon, both the good contractility and wettability of PET and the good flexibility of nylon are secured, thus providing a good wearing sensation as a sheet for a mask pack to a user.

In order to secure adequate air permeability and elasticity for the sheet for the mask pack and also more stably split the split yarn to a high degree of splitting, the split yarn is an N/P split yarn which is formed from the thermoplastic resin including 60 to 80 wt% of polyethylene terephthalate (PET) and 20 to 40 wt% of nylon. Further, the split yarn may be one or more types selected from among eight-split yarns, sixteen-split yarns, and thirty-two-split yarns. Among them, sixteen-split yarns or thirty-two-split yarns are preferable, and the case where the proportion of thirty-two-split yarns is high, if possible, is more preferable for securing flexibility of the sheet by achieving ultrafine yarns.

In the present invention, the split yarn is preferably split into microfine fibers having an average diameter of 0.01 to 2 µm per strand of fiber after being split. Of course, there is a conventional technology for manufacturing a nonwoven sheet for a mask pack using microfine fibers, disclosed in Korean Patent Application KR 2011-0060525. However, in the above-described conventional technology, a melt-blown method of forming microfine fibers by performing stretching while high-pressure hot air is applied when filaments are discharged from a spinneret is applied. However, since the microfine-fiber nonwoven fabrics manufactured using the melt-blown method have very low strength, the fabrics are easily torn or exhibit insufficient supporting force when attached to the skin.

On the other hand, the nonwoven sheet for the mask pack of the present invention may be manufactured using a spun-bond method. According to the spun-bond method, after short staple fibers are formed using the spun filaments, the short staple fibers may be scattered in the form of a web and heat may be applied thereto to thus bond the fibers forming the web, thereby manufacturing a nonwoven fabric. Accordingly, good mechanical properties (tensile strength) may be secured compared to nonwoven fabrics manufactured using the melt-blown method. That is, since the nonwoven sheet for the mask pack of the present invention is manufactured using the spun-bond method, the basic strength of the nonwoven sheet may be achieved. Further, since the split yarn is used, the nonwoven sheet that is finally manufactured may include microfine fibers having an average diameter of 0.5 to 2 µm, whereby superior skin adhesion characteristics and tactile softness may be secured.

With respect to the constitution in which the nonwoven sheet for the mask pack of the present invention is manufactured using the spun-bond method and includes microfine fibers, there is another technical meaning. Generally, in the manufacture of the nonwoven fabric using the spun-bond method, there is a drawback in that it is difficult to apply the method when the average diameter of the fibers is 10 µm or less. However, since the split yarn is applied in the present invention, the microfine fibers may be included in the nonwoven fabric manufactured using the spun-bond method.

A process for manufacturing the nonwoven sheet for the mask pack of the present invention may be more specifically described as follows.

First, two or more types of thermoplastic resin are put into separate extruders and then melted, and two or more types of melted resins meet with each other in a spinneret designed to include a nozzle having a specific shape (for example, a radial type). Thereby, split yarn including two or more types of thermoplastic resin in one filament is spun. The spun split yarn is sufficiently stretched using a high-pressure air-stretching device to thus manufacture filament fibers having a diameter of 10 to 25 µm, which is the average diameter that may be typically applied to a spun-bond process.

The stretched filaments are opened according to a typical spun-bond process, are layered in the form of a web, and are thermally bonded to each other using a high-temperature and high-pressure calender roll or embossing roll. Thereafter, physical external force is applied to the manufactured nonwoven fabric using a high-pressure water jet, and during this process, physical bonds between the two types of polymers forming the split yarn are broken and the polymers are split into microfine yarns due to the lack of mutual compatibility (affinity). The pressure of the high-pressure water jet may be applied to a level of 50 to 280 bar, and the split yarn may be split into microfine yarns to thus further complicate the structure of the web, thereby further improving the strength of the nonwoven fabric. If the pressure of the water jet is 50 bar or less, complete splitting into microfine yarns is not performed, and only hairness is formed on the surface, so the degree of splitting is insignificant. Accordingly, it is difficult to secure softness and strength and to apply the nonwoven fabric to the sheet for the mask pack due to the hairness formed on the surface.

In the case where the split yarn includes polyethylene terephthalate (PET) in the present invention, in addition to the physical splitting process using the water jet, a PET component may be further dissolved in an amount of 10 to 20% using an alkali aqueous solution (for example, NaOH), whereby filaments that have not been physically split may be chemically split. Particularly, since dissolution of PET using the alkali aqueous solution may impart increased softness to PET, a method for achieving a soft texture on the final mask pack sheet may be preferably applied.

The nonwoven sheet for the mask pack of the present invention preferably has a unit weight of 30 to 60 g/m² on a dry-weight basis, a thickness of 0.15 to 0.3 µm, and a porosity of 60 to 95%. When such a condition is satisfied in the manufacture, a greater quantity of coarse cosmetic solutions (such as a functional drug) may be carried in the space between the fibers, and the sheet may stick well to the skin without feeling uncomfortable after being applied on the face.

### Mode for Invention

### Example

Hereinafter, the present invention will be described in more detail with reference to Examples.

### Example 1

Polyethylene terephthalate (Kolon Company, P-25 SDT (Semi Dull Grade)), as a first polymer, and nylon (DOMO Company, NY6 CHIP Domamid 2403T-HS), as a second polymer, were each melted at a spinning temperature of 288°C using a continuous extruder, and then complex spinning was performed while the ratio of the content (wt%) of the first polymer and the second polymer was set to 70 : 30. Subsequently, the continuous filaments discharged from a capillary opening were solidified using blown air for cooling and then stretched at a spinning speed of 5000 m/min using a high-pressure air-stretching device to thus manufacture thirty-two-split yarns having a filament average diameter before splitting of 17.32 µm. The manufactured filament fibers were layered in the form of a web on a conveyer net using a typical opening method, and then subjected to a calendering process using a calender roll heated to a temperature of about 130°C to thus manufacture spun-bond nonwoven fabrics having smoothness, a suitable thickness, and a weight of 45 g/m² (on a dry-weight basis).

Thereafter, pressure was applied to the nonwoven fabrics in the order of intensities of lower (80 bar)-upper (280 bar)-lower (60 bar) pressures using a water jet. The application of the pressure in the order of the lower-upper-lower pressures is performed so that, after the shape of the nonwoven sheet is fixed using initial confounding at a low pressure, splitting and confounding of the filaments are performed at a high pressure, and the smoothness of the filaments is increased at a low pressure to thus prevent the filaments from agglomerating. When the initial pressure is excessively high, wrinkles or holes may be formed in the nonwoven sheet. When the middle-stage pressure of the water jet is excessively low or high, it may be difficult to exhibit proper strength. Finally, the intensity of end pressure determines the quality of nonwoven fabrics (apparent shape), so it is important to suitably control the intensity of pressure.

Subsequently, a portion of the PET polymer was dissolved using an alkaline solvent (NaOH) so that a weight loss ratio is about 15 to 20% (weight loss), thus increasing the degree of splitting of the split-yarn filaments and improving the tactile softness. After the above processing, washing and drying processes were finally performed to thus manufacture spun-bond nonwoven fabric materials for a mask pack sheet.

### Example 2

Example 2 was manufactured using the same method as in Example 1, except that the middle-stage pressure of the water jet was changed to 150 bar.

### Example 3

Example 3 was manufactured using the same method as in Example 1, except that the weight loss ratio was changed to 10%.

### Example 4

Example 4 was manufactured using the same method as in Example 1, except that the filament was manufactured so as to include sixteen-split yarns.

### Reference Example 5 (not according to the invention)

### Example 5

Reference Example 5 was manufactured using the same method as in Example 1, except that complex spinning was performed while the ratio of the contents (wt%) of the first polymer (PET) and the second polymer (nylon) was set to 90 : 10.

### Example 6

### Reference Example 6 (not according to the invention)

Reference Example 6 was manufactured using the same method as in Example 1, except that complex spinning was performed while the ratio of the contents (wt%) of the first polymer (PET) and the second polymer (nylon) was set to 50 : 50.

### Comparative Example 1

Filaments were manufactured using only polyethylene terephthalate (Kolon Company, P-25 SDT (Semi Dull Grade), used as the first polymer in Example 1, were layered in the form of a web on a conveyor net, and were subjected to a calendering process using a calender roll heated to a temperature of about 130°C to thus manufacture spun-bond nonwoven fabric (Comparative Example 1) having a weight of 45 g/m². Further, splitting and alkali weight-loss processes were performed using a water jet according to the same method as in Example 1.

### Comparative Example 2

Filaments were manufactured using only nylon (DOMO Company, NY6 CHIP Domamid 2403T-HS), used as the second polymer in Example 1, were layered in the form of a web on a conveyor net, and were subjected to a calendering process using a calender roll heated to a temperature of about 130°C to thus manufacture spun-bond nonwoven fabrics (Comparative Example 2) having a weight of 45 g/m². Further, splitting and alkali weight-loss processes were performed using a water jet according to the same method as in Example 1.

### Comparative Example 3

Comparative Example 3 was manufactured using the same method as in Example 1, except that sea-island type PET/CoPET filaments (Kolon Company) including PET (island component) and CoPET (sea component) at a component ratio of 6 : 4 were used instead of N/P split yarns as raw yarns and that fibers were made thin using only a method (weight loss) of dissolving 40% of the sea component with an alkaline solvent (NaOH).

### Comparative Example 4

PET and nylon were prepared at a weight ratio of 70 : 30 and were put into two extruders. Subsequently, air at a pressure of 5 psi and a temperature of 250°C was supplied while spinning was performed, and a cooling temperature was set to 10°C, thus manufacturing melt-blown nonwoven fabric (Comparative Example 4) having a weight of 45 g/m².

### Measurement Example I

The air permeability, the degree of splitting, the fiber pore size, the strength, the elongation, the density, and the flexibility of the nonwoven sheets for mask packs of Examples 1 to 3 that were manufactured were measured using the following method, and the results are described in the following Table 1.

### <Method of measuring physical properties>

(1) Air permeability: air permeability was measured using the Frazier test method according to an ASTM D 737 regulation. Specifically, the amount of air (cm³/cm²/sec) flowing into a specimen having a size of 38 cm² at a drain pressure of 125 Pa was obtained to thus measure the air permeability.
(2) Degree of splitting: the degree of splitting was calculated using ten pictures (10 ea) obtained by photographing the sections of the nonwoven fabrics using a microscope with a magnification x200 according to the following Equation 1. (Number of split filaments) / (Number of filaments) * 100
(3) Fiber pore size: after Galwick fluid (a substance having a surface tension lower than that of water) was impregnated on a specimen of 3 cm in diameter, air was flowed at a constant speed while the air pressure was increased, and the pressure change was measured (Measurement equipment: PMI capillary flow porometer) and calculated, thereby measuring pore sizes between the filaments.
(4) Strength and elongation: A KS K 0521 method was used for tensile strength and tensile elongation. Specifically, after a nonwoven specimen having a size of 5 × 20 cm in width and length was held with a 5 × 5 cm jig at the top and bottom thereof, measurement was performed at a tensile speed of 200 mm/min using measurement equipment manufactured by the INSTRON company.
(5) Sleekness (MIU): The sleekness of the surface was measured using the top, bottom, left, and right fluctuation according to the surface roughness while a piano string was moved on the surface of the nonwoven fabric in the state of being into contact with the surface of the nonwoven fabric (Measurement equipment: KES-SE/KATO TECH Co., LTD/Japan). It was judged that the smaller the value, the sleeker the surface.
(6) Flexibility (stiffness): A panel was provided at an angle of 45° on the edge of a testing bench which was parallel to the bottom surface. The nonwoven sheet was pushed at a constant speed toward the edge of the testing bench, and the length of a portion of the nonwoven sheet extending outwards from the edge of the testing bench was measured on the basis of the time point when the nonwoven sheet reached the panel. It can be read that the longer the extension length of the nonwoven sheet, the lower the flexibility.

**[Table 1]**

| | Air permeability (ccs) | Degree of splitting | Fiber pore size | Strength (kgf/5 cm) | Elongation (%) | Sleekness (MIU) | Flexibility (stiffness) |
|---|---|---|---|---|---|---|---|
| Example 1 (Thirty-two-split yarns) | 30 | 90% | 8 to 10 µm | 11 | 30 | 0.28 µm | 3.0 cm |
| Example 2 (Thirty-two-split yarns) | 50 | 68% | 14 to 20 µm | 8 | 36 | 0.34 µm | 2.8 cm |
| Example 3 (Thirty-two-split yarns) | 45 | 70% | 12 to 18 µm | 10 | 25 | 0.34 µm | 2.5 cm |
| Example 4 (Sixteen-split yarns) | 64 | 72% | 17 to 23 µm | 12 | 28 | 0.48 µm | 3.8 cm |
| Ref. Example 5 (N/P:1/9) | 62 | 40% | 24 to 28 µm | 12 | 31 | 0.39 µm | 4.2 cm |
| Ref. Example 6 (N/P:5/5) | 58 | 56% | 22 to 27 µm | 11 | 32 | 0.41 µm | 2.2 cm |
| Comparative Example 1 (PET) | 82 | 9% | 34 to 37 µm | 16 | 41 | 0.53 µm | 4.6 cm |
| Comparative Example 2 (Nylon) | 75 | 12% | 30 to 38 µm | 12 | 38 | 0.51 µm | 5.3 cm |
| Comparative Example 3 (Sea-island yarn) | 24 | 100% | 5 to 7 µm | 4 | 18 | 0.21 µm | 1.6 cm |
| Comparative Example 4 (Melt-blow) | 22 | - | 4 to 6 µm | 2 | 13 | 0.19 µm | 1.2 cm |

The nonwoven sheets for the mask pack of Examples 1 to 4 manufactured according to the present invention and Reference Example 5 had the good air permeability and physical properties due to the formation of micropores and were sleek and flexible, so they were evaluated to adhere more strongly to the skin when applied as a sheet for a mask pack and to provide a good sensation when used. Particularly, Examples 1 to 3, in which the N/P ratio was 3/7 and which used thirty-two-split yarns, exhibited good physical properties and excellent flexibility.

### Measurement Example II. Blind test

Usable mask packs were manufactured using the nonwoven sheets of Examples 1 to 4, Reference Examples 5 and 6, and Comparative Examples 1 to 4. A blind test was further performed in order to check actual user satisfaction with the use of the sheet. One of the essences sold on the market was randomly selected and prepared in an amount of 20 mL. The nonwoven sheets of Examples 1 to 4, Reference Examples 5 and 6, and Comparative Examples 1 to 4 were each punched out in the form of a mask pack, and then carried in the prepared essence for 1 hour to thus manufacture a mask pack.

20 male panelists and 20 female panelists (40 total) in each of their teens, 20s, 30s, 40s, and 50s were selected as subjects for the experiment. The panelists were instructed to use 9 types of prepared mask packs over 9 days, regardless of the order thereof (usage time was limited to 20 minutes). The subjects of the experiment were asked to evaluate initial adhesion, supporting force (flowing down during use), persistence, and texture on attachment after using the mask pack according to the following evaluation criteria. The subjects' responses were averaged (Discard less than two decimal places), and are shown in Table 2.

### <Evaluation criteria>

(1) Initial adhesion: When the mask pack was attached to the face, the degree of adhesion of the sheet to the skin along the curves of the face was evaluated based on a 5-point scaling
   - Initial adhesion score of 5-point scaling

Very good: 5 points / Good: 4 points/ Average: 3 points/ Dissatisfactory: 2 points / Very dissatisfactory: 1 point
(2) Supporting force: When the mask pack carrying the essence was attached in everyday life, the degree to which the mask pack flowed down from the face due to the weight of the essence was evaluated based on a 5-point scaling

### - Supporting force score of 5-point scaling

No disruption to activities: 5 points / Similar to normal mask packs (no problem with moving to nearby areas): 4 points / Less moving activities possible: 3 points / Feeling uncomfortable upon removal of packs from face : 2 points / Moving not possible: 1 point

(3) Persistence: The time point at which the essence carried in the nonwoven sheet was absorbed by the skin and at which the nonwoven sheet began to dry after 20 minutes of use was evaluated based on a 5-point scaling

### - Persistence score of 5-point scaling

Moisture retained when worn: 5 points / Sheet begins to dry after 18 minutes: 4 points / Sheet begins to dry at 16 to 17 minutes: 3 points / Sheet begins to dry at 13 to 15 minutes: 2 points / Sheet begins to dry in 12 minutes or less: 1 point

(4) Texture on attachment: When the mask pack was attached to the face, the texture on attachment to the skin was evaluated based on a 5-point scaling

### - Texture-on-attachment score of 5-point scaling

Very good: 5 points / Good: 4 points /Average: 3 points / Dissatisfactory: 2 points / Very dissatisfactory: 1 point

**[Table 2]**

| | Example 1 | Example 2 | Example 3 | Example 4 | Ref. Example 5 | Ref. Example 6 | Comparative Example 1 | Comparative Example 2 | Comparative Example 3 | Comparative Example 4 |
|---|---|---|---|---|---|---|---|---|---|---|
| Initial adhesion | 4.7 | 4.8 | 4.7 | 3.8 | 3.7 | 4.3 | 2.0 | 1.8 | 4.8 | 4.8 |
| Supporting force | 4.2 | 4.1 | 4.1 | 3.8 | 3.9 | 3.9 | 2.9 | 2.8 | 1.7 | 1.8 |
| Persistence | 4.9 | 4.8 | 4.9 | 4.2 | 3.7 | 3.9 | 2.1 | 1.7 | 4.7 | 4.8 |
| Texture on attachment | 4.9 | 4.9 | 4.9 | 4.2 | 4.3 | 4.5 | 2.0 | 1.8 | 4.9 | 4.9 |

As a result of the blind test, Comparative Examples 1 and 2 were evaluated to be 2 points or less in initial adhesion, and Comparative Examples 1 to 4 were evaluated to be less than 3 points in supporting force. Comparative Examples 1 and 2 were evaluated to be 2 points or less in persistence and texture on attachment. Comparative Examples 3 and 4 were 4.0 or more and evaluated to be good in all of initial adhesion, persistence, and texture on attachment, but were evaluated to be lowest in supporting force.

On the other hand, in the case of Examples 1 to 4, manufactured using the split yarns according to the present invention, and Reference Examples 5 and 6, the score was 3.0 or more in all the evaluations, and in particular, Examples 1 to 3 were 4.0 or more, and evaluated to be very good.

## Claims

1. Use of a nonwoven sheet as a mask pack sheet, wherein the nonwoven sheet comprises:
split yarns formed from two or more types of thermoplastic resin, wherein the two or more types of thermoplastic resin include polyethylene terephthalate (PET) and nylon,
wherein the split yarns are formed from the thermoplastic resin including 60 to 80 wt% of the polyethylene terephthalate (PET) and 20 to 40 wt% of the nylon.

2. The use of the nonwoven sheet of claim 1 wherein the split yarns are one or more types selected from among eight-split yarns, sixteen-split yarns, and thirty-two-split yarns.

3. The use of the nonwoven sheet of claim 2, wherein in the split yarns, an average diameter per strand of fiber after being split is 0.01 to 2 µm.

## Patentansprüche

1. Verwendung eines Vliesstofftuchs als ein Maskenpackungstuch, wobei das Vliesstofftuch Folgendes umfasst:
Spaltgarne, die aus zwei oder mehr Arten von thermoplastischem Harz ausgebildet sind, wobei die zwei oder mehr Arten von thermoplastischem Harz Polyethylenterephthalat (PET) und Nylon einschließen,
wobei die Spaltgarne aus dem thermoplastischen Harz, das 60 bis 80 Gew.-% des Polyethylenterephthalats (PET) und 20 bis 40 Gew.-% des Nylons einschließt, ausgebildet sind.

2. Verwendung des Vliesstofftuchs nach Anspruch 1, wobei die Spaltgarne eine oder mehrere Arten, die aus acht-fädigen Spaltgarnen, sechzehn-fädigen Spaltgarnen und zweiunddreißig-fädigen Spaltgarnen ausgewählt sind, sind.

3. Verwendung des Vliesstofftuchs nach Anspruch 2, wobei in den Spaltgarnen ein durchschnittlicher Durchmesser pro Faserstrang nach dem Spalten 0,01 bis 2 µm beträgt.

## Revendications

1. Utilisation d'une feuille non tissée comme une feuille d'ensemble masque, dans lequel la feuille non tissée comprend :
des fils fendus formés à partir d'au moins deux types de résine thermoplastique, les au moins deux types de résine thermoplastique comportant du polyéthylène téréphtalate (PET) et du nylon,
dans laquelle les fils fendus sont formés à partir de la résine thermoplastique comprenant 60 à 80 % en poids de polyéthylène téréphtalate (PET) et 20 à 40 % en poids de nylon.

2. Utilisation de la feuille non tissée selon la revendication 1, dans laquelle les fils fendus sont d'un ou plusieurs types choisis parmi des fils fendus en huit, des fils fendus en seize et des fils fendus en trente-deux.

3. Utilisation de la feuille non tissée selon la revendication 2, dans laquelle, dans les fils fendus, un diamètre moyen par brin de fibre après avoir été fendu est de 0,01 à 2 µm.
